# EUROPÄISCHE PATENTSCHRIFT

(11) **EP 1 561 482 B1**
(45) Veröffentlichungstag und Bekanntmachung des Hinweises auf die Patenterteilung: **18.10.2006**
(21) Anmeldenummer: 04004955.3
(22) Anmeldetag: 07.02.2004
(51) Int. Cl.: A61M 5/00, A61M 5/14

(54) **Verfahren zur Vorbereitung eines Sets zur intrathekalen Verabreichung von Zytostatika-/Medikamenten-Lösungen**
Process for the preparation of one set for intrathecal administration of solutions of cytostatic drugs
Procédé pour la préparation d'un set pour l'administration intrathécale de solutions de medicaments cytostatiques

(43) Veröffentlichungstag der Anmeldung: 10.08.2005
(73) Patentinhaber: Codan Holding GmbH, 23738 Lensahn (DE)
(72) Erfinder: Crombach, Josef J.M., 8251 VH Dronten (NL)
(74) Vertreter: Fleck, Thomas

(56) Entgegenhaltungen:
- EP-A- 0 430 398
- EP-A- 0 992 257
- US-A- 4 871 353
- US-A1- 2003 159 741
- US-B1- 6 500 153

## Beschreibung

Die Erfindung betrifft ein Verfahren zur Vorbereitung eines Sets für die sichere Verabreichung von Zytostatika-/Medikamenten-Lösungen in den intrathekalen Bereich eines Patienten.

Aus der EP 0 992 257 A1 ist eine einen Infusionsfilter aufweisende Ventilbank zur Infusion verschiedener Flüssigkeiten bekannt, wobei die Ventilbank mehrere in Aufsätzen angeordnete Rückschlagventile aufweist, die mit Medikamentenspritzen verbunden werden können. Aus der US 4,871,353 ist ein Übertragungsschlauch zur Verabreichung einer isotonischen NaCl-Lösung während einer Operation bekannt, der standardisierte Luer-Lock-Anschlüsse zum Einspritzen ergänzender Medikamente besitzt. Im weiteren Stand der Technik ist aus der US 6,500,153 eine Spinalkanüle mit Spritze bekannt, die zur Verabreichung eines Wirkstoffs dient, der spinal in den Patienten injiziert wird.

Ziel der Erfindung ist es, Verwechslungen von Zytostatika-/Medikamentenspritzen in den intrathekalen Bereich auszuschließen. In den letzten Jahren führten solche Verwechslungen von Zytostatika-/Medikamentenspritzen, die eigentlich intravenös verabreicht werden sollten, jedoch an intrathekale Zugänge angeschlossen und verabreicht wurden, zum Tod der Patienten. Mit diesem Problemenbereich beschäftigt sich auch schon die WO 02/22201 A1, deren Lösungsvorschläge jedoch auf besonders gestalteten Kupplungsstücken beruhen.

Aus mehreren Ländern wurden in den vergangenen Jahren derartige Verwechslungen von Zytostatika-/Medikamentenspritzen berichtet. Bei dieser bestimmten onkologischen Therapieform erhält der Patient die Zytostatika-/Medikamentenspritzen annähernd zeitgleich über den intrathekalen und intravenösen Zugang. Die Zytostatika/Medikamente sind unterschiedlich und dürfen nur über den bestimmten Zugang verabreicht werden. Als intrathekaler Zugang wird in der Regel eine Spinalkanüle mit Luer-Lock negativ Anschluß verwendet, der intravenöse Zugang ist in der Regel eine peripher gelegte Venenverweilkanüle, auch mit Luer-Lock negativ Anschluß.

Die zu verabreichenden Zytostatika-/Medikamentenspritzen mit Luer-Lock Anschluß werden in der Krankenhausapotheke vorbereitet, gekennzeichnet und dann an den Applikationsort geliefert. Dort erfolgt die Verabreichung der Medikamente durch den behandelnden Arzt. Durch Unachtsamkeit, Streß und Hektik fanden hier die Verwechslungen der Spritzen statt, häufig so, daß die intravenös zu verabreichenden Zytostatika/Medikamente intrathekal verabreicht wurden. Diese Fälle führten dann zum Tod der Patienten.

Die Aufgabe der Erfindung ist deshalb, solche schwerwiegenden Verwechslungen in Zukunft auszuschließen und zusätzlich den Anwendern eine kontaminationssichere Arbeitsweise zur Verfügung zu stellen.

Diese Aufgabe wird in überraschender Weise durch das Verfahren nach Anspruch 1 gelöst.

Durch das Vorsehen mehrerer Zytostatika-/Medikamentenspritzen, ihr vorheriges Füllen und Anschließen und das nachträgliche Spülen des Systems mit der Kochsalzlösung - zumal in der Apotheke der Set auf Richtigkeit geprüft wird - besteht eine signifikant geringere Gefahr der Kontamination der Anwender und der Patienten durch die Zytostatika/Medikamente, als im Vergleich im Umgang mit Einzelspritzen, wobei Verwechslungen nicht ausgeschlossen sind, die an den Zugang konnektiert und nach Entleerung wieder entkonnektiert werden müssen.

Ein bevorzugtes Ausführungsbeispiel, auf das die Erfindung nicht beschränkt ist, wird anhand der Zeichnung im folgenden zum besseren Verständnis der Erfindung näher erläutert. Es zeigt:

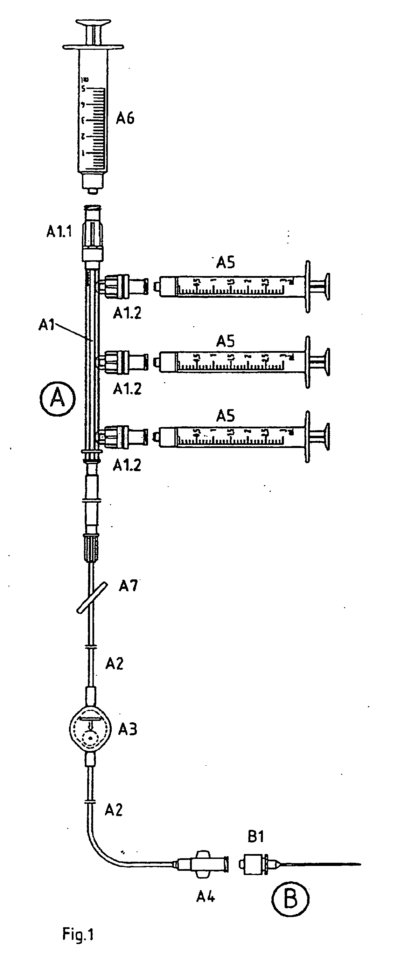

- Fig. 1: ein eingesetztes Set in seinem schematischen Aufbau mit seinen Einzelteilen vor ihrem Zusammenbau.

Das Set A mit der integrierten Ventilbank A1 und den integrierten Ventilen A1.1, A1.2 und dem Übertragungsschlauch A2 mit Infusionsfilter A3 wird überwiegend in der Krankenhausapotheke vorbereitet, wozu die drei (zwei oder mehr) Zytostatika-/Medikamentenspritzen A5, sowie eine Kochsalzlösungsspritze A6 an das Ventil A1.1 über die Luer-Lock Anschlüsse fest angeschlossen werden. Mit der Kochsalzlösungsspritze A6 werden die Ventilbank A1, der Übertragungsschlauch A2 und der Infusionsfilter A3 und der Anschluß A4 entlüftet und mit der Schlauchklemme A7 wird der Übertragungsschlauch A2 während des Transportes verschlossen. Anschließend wird die Einheit bzw. das Set gekennzeichnet, geprüft und verpackt und an den Applikationsort geliefert. Dort erhält der Patient durch den Arzt eine spezielle Spinalkanüle B mit Luer-Lock positiv Anschluß B1 in den intrathekalen Bereich gelegt. Anschließend wird eine Menge Liquorflüssigkeit in einem Behältnis aufgefangen, woraufhin das gekennzeichnete und vorbereitete Set A mit den konnektierten Spritzen A5 und A6 mit dem Luer-Lock negativ Anschluß A4 an die Spinalkanüle B mit dem Luer-Lock positiv Anschluß B1 konnektiert wird.

Im Anschluß erfolgt die Verabreichung der Zytostatika-/Medikamentlösungen nacheinander sowie die abschließende Spülung des Systems mit der Kochsalzlösung nach der Medikamentengabe. Kleine Luftmengen sowie Partikel die bei der Vorbereitung und Verabreichung auftreten können, werden durch den Infusionsfilter A3 sicher zurückgehalten. Das integrierte Ventil A1.1 verhindert einen Rückfluß/Rückförderung von Flüssigkeiten in die Spritze A6. Die Zytostatika-/Medikamentenspritzen A5 werden durch drucköffnende Ventile A1.2 gesichert, so daß es nicht zum Selbstentleeren der Spritzen durch Schwerkraftantrieb kommen kann. Die Ventilbank A1 mit dem Übertragungsschlauch A2 mit Infusionsfilter A3 und Anschluß A4 hat ein geringes Füllvolumen von beispielsweise ≤ 2.2 ml.

Die spezielle Spinalkanüle B mit Luer-Lock positiv Anschluß erlaubt nur den Anschluß des vorbereiteten Sets A. Der Anschluß von herkömmlichen Einmalspritzen mit Luer-Lock positiv Anschlüssen ist nicht möglich. Eine Verwechslung am Applikationsort der Zytostatika-/Medikamentenspritzen, d.h. die Falschgabe von Zytostatika/Medikamenten in den intrathekalen Bereich, kann somit bei dieser Anwendung nicht mehr vorkommen.

Beschreibt wird also den Einsatz eines kompletten, geschlossenen Set (Kit) mit allen notwendigen Komponenten für die sichere intrathekale Zytostatika-/Medikamentenverabreichung, bestehend aus einem "Ventilbanksystem" mit Luer-Lock negativ Anschluß, der Spinalkanüle mit Luer-Lock positiv Anschluß und den Einmalspritzen das auf kostenintensive Komponenten, die außerhalb von Standards liegen, verzichtet.

## Patentansprüche

1. Verfahren zur Vorbereitung eines Sets für die sichere, kontaminationsfreie Verabreichung von Zytostatika/Medikamenten-Lösungen in den intrathekalen Bereich eines Patienten, wobei das Set folgende Teile umfasst:
a) einen Übertragungsschlauch (A2) mit einer Schlauchklemme (A7) und standardisierten Luer-Lock Anschlüssen, von denen ein distaler Anschluss als Luer-Lock negativ Anschluss (A4) ausgebildet ist;
b) einen Infusionsfilter (A3);
c) eine Ventilbank (A1) mit verschieden wirkenden Ventilen und Luer-Lock-Anschlüssen;
d) mehrere ausgewählte Zytostatika-/Medikamentenspritzen (A5); und
e) eine NaCl-Lösung enthaltende Spritze;
und wobei das Verfahren folgende Schritte umfasst :
a') Anschließenden mehreren ausgewählten Zytostatika-/Medikamentenspritzen (A5), der NaCl-Lösung enthaltenden Spritze (A6) über Luer-Lock-Anschlüsse an die Ventilbank (A1) mit Ventilen (A1.1 und A1.2),
b') Entlüften der Ventilbank (A1), des Übertragungsschlauches (A2), des Infusionsfilters (A3) und des Luer-Lock negativ Anschlusses (A4), Schließen der Schlauchklemme (A7),
c') Kennzeichnen, Prüfen und Verpacken des Sets als Einheit und Beilegen einer Spinalkanüle (3) mit Luer-Lock positiv Anschluss (B1),
d') Liefern desselben zum Applikationsort.

## Claims

1. Process for the preparation of a set for the safe, contamination-free administration of cytostatic/medicament solutions to the intrathecal area of a patient, in which the set comprises the following parts:
a) a transfer tube (A2) having a tube clamp (A7) and standardized Luer Lock connections, whereof a distal connection is constructed as a Luer Lock negative connection (A4),
b) an infusion filter (A3),
c) a valve bank (A1) with differently acting valves and Luer Lock connections,
d) several selected cytostatic/medicament syringes (A5) and
e) a syringe containing NaCl solution,
and in which the process comprises the following steps:
a') the connection of several selected cytostatic/medicament syringes (A5) and the NaCl solution-containing syringe (A6) via Luer Lock connections to the valve bank (A1) having valves (A1.1 and A1.2),
b') venting the valve bank (A1), transfer tube (A2), infusion filter (A3) and the Luer Lock negative connection (A4) and closing the tube clamp (A7),
c') marking, inspecting and packing the set as a unit and enclosing a spinal cannula (3) with a Luer Lock positive connection (B1),
d') supplying the same to the application location.

## Revendications

1. Procédé de préparation d'un jeu pour l'administration en toute sécurité et sans contamination de solutions médicamenteuses cytostatiques dans la zone intrathécale d'un patient, le jeu comprenant les éléments suivants :
a) un flexible de transmission (A2) avec un clamp tubulaire (A7) et des raccords Luer-Lock standardisés dont un raccord distal est configuré comme un raccord négatif Luer-Lock (A4) ;
b) un filtre de perfusion (A3) ;
c) un banc de valves (A1) avec des valves à actions variables et des raccords Luer-Lock ;
d) plusieurs seringues de médicaments cytostatiques sélectionnées (A5) ; et
e) une seringue contenant une solution NaCl ;
et le procédé comprenant les étapes suivantes :
a') raccord de la pluralité de seringues de médicaments cytostatiques sélectionnées (A5), de la seringue (A6) contenant la solution NaCl par des raccords Luer-Lock au banc de valves (A1) avec des valves (A1.1 et A1.2),
b') évacuation de l'air du banc de valves (A1), du flexible de transmission (A2), du filtre de perfusion (A3) et du raccord négatif Luer-Lock (A4), fermeture du clamp tubulaire (A7),
c') identification, contrôle et conditionnement du jeu en tant qu'unité et ajout d'une canule spinale (3) avec raccord positif Luer-Lock (B1),
d') livraison de celui-ci vers le site d'application.
